# EUROPEAN PATENT SPECIFICATION

(11) **EP 1 545 294 B1**
(45) Date of publication and mention of the grant of the patent: **27.02.2008**
(21) Application number: 03732603.0
(22) Date of filing: 17.06.2003
(51) Int. Cl.: A61B 3/16

(54) **A METHOD FOR MEASURING INTRAOCULAR PRESSURE**
VERFAHREN ZUR MESSUNG DES AUGENINNENDRUCKS
PROCEDE DESTINE A MESURER LA TENSION INTRA-OCULAIRE

(30) Priority: 17.06.2002 FI 20021172
(43) Date of publication of application: 29.06.2005
(73) Proprietor: Tiolat Oy, 00660 Helsinki (FI)
(72) Inventor: KONTIOLA, Antti, FIN-00660 Helsinki (FI)
(74) Representative: Söderman, Päivi Karin Lisbeth
(86) International application number: PCT/FI2003/000490
(87) International publication number: WO 2003/105681

(56) References cited:
- WO-A1-96/06560
- US-A- 5 176 139
- US-A- 6 093 147
- US-B1- 6 394 954

## Description

The present invention relates to a method for measuring intraocular pressure. The method and the apparatus used for the same are based on an understanding of the laws governing the impact of an object with the eye.

Intraocular pressure is generally measured using a tonometer, which is placed on the surface of the cornea and which measures its elasticity using various methods (Goldmann's tonometer, Schiötz's tonometer, etc.). Two of the most commonly used principles are the measurement of the force required to applanate a certain area of the surface of the eye, or the measurement of the diameter of the area that is applanated by a known force. These methods require the patient's cooperation and cannot be applied without general anaesthesia to small children, persons suffering from dementia, or animals.

Methods, such as US patent publications, 5148807, 5279300, and 5299573, in which the surface of the cornea is not touched, the intraocular pressure being measured instead with the aid of a water or air jet, or various kinds of waves, have also been developed. These methods are technically complex and thus expensive. Meters operating on the air-jet principle are widely used by opticians, but their cost has prevented them from being more extensively used by general practitioners.

US patent publication 5176139 also discloses a method, in which a freely-falling ball is dropped onto the eyelid and the height of the ball's rebound is measured.

It is also known from Finnish patent application 973094 an apparatus which is based on the fact that a probe is forwarded with a certain speed to contact the surface of the eye, wherefrom it will rebounded. The movements of the probe can be the basis for calculation of the intraocular pressure.

Document US-A-6 093 147 discloses a method for measuring intraocular pressure using a probe within a case which is accelerated towards the eye by feeding a short constant voltage pulse to a coil surrounding the probe.

The present invention is intended to achieve an improved method specifically when using the latter quite good apparatus. The intention is also, however, to retain the apparatus's simple, economical, and precisely measuring basic construction, by means of which intraocular pressure can also be measured in patients incapable of co-operating, who can be restrained only momentarily. In addition, the meter is also suitable for extensive screening campaigns, as measurement is rapid and requires neither a local anaesthetic nor specially trained operators. It is also intended to permit home monitoring for patients with intraocular pressure complaints.

The above and other benefits and advantages are achieved by the method according to the invention which is defined in claim 1.

In the following, the invention is examined with reference to the accompanying drawings, showing the apparatus used in connection of the method according to the invention, in which drawings certain well regarded properties of the invention are presented.

Thus:
Figure 1 shows one version of a practical apparatus, in order to provide a general view of it;
Figure 2 shows a vertical cross section of the above, rotated to an angle of 90 degrees to the above;
Figure 3 shows a more detailed picture of the essential component of the invention, by means of which the probe is launched towards the eye.

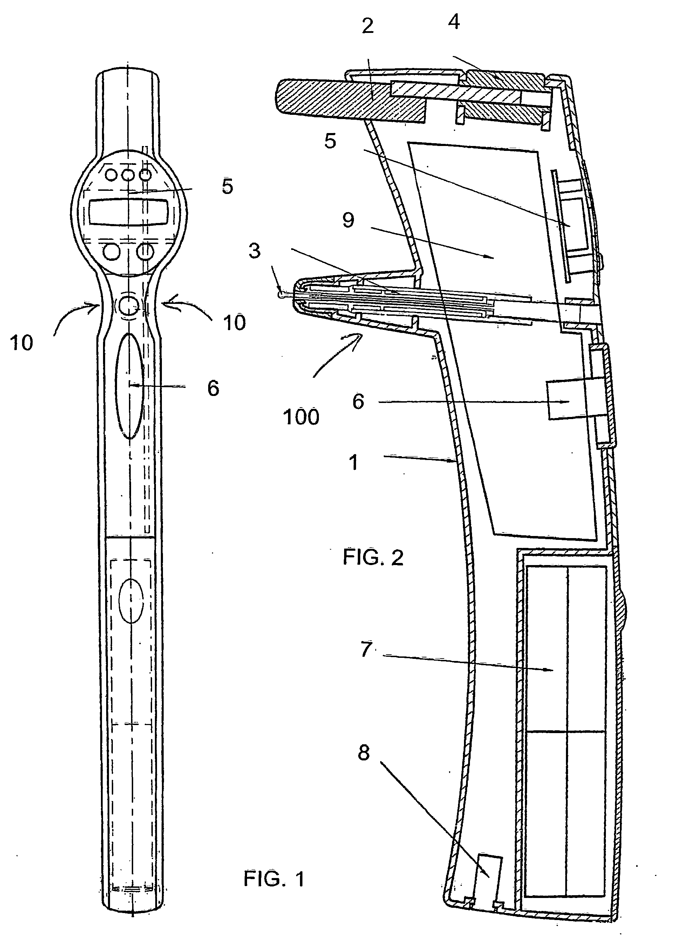

Thus, Figure 1 shows, as stated above, one practical embodiment of an apparatus applying the principle according to the invention, while Figure 2 shows a cross section of it. These two figures can be used together to illustrate the general principle and construction.

The apparatus is formed of a case component 1 made of a suitable material, inside of which all the components that are essential for measurement are fitted.

The case or body component 1 is essentially elongated and includes at its upper end a forehead support 2, which is intended to adjust the distance from which the probe 3 impacts the eye being measured. The forehead support 2 is specifically adjustable, one comfortable way of arranging adjustability being to use an adjustment wheel 4, which can be easily rotated with a finger.

The apparatus includes a display and control component 5, which is particularly a liquid-crystal panel, in which the measurement result is displayed, and the related control buttons, etc, which are required at various times. Reference number 6 marks the operating switch, which, when pressed, releases the probe 3 towards the eye.

Operating power for the apparatus comes from dry cells or batteries 7, while the apparatus can additionally have a socket 8, to which an external recharging device or power supply can be connected.

The electronics of the apparatus according to the invention are assembled on a circuit board 9, which is shown schematically.

Figure 1 clearly shows one property of the apparatus according to the invention that makes it more comfortable and accurate to use. This is the narrowed part, incorporated in the apparatus next to the probe 3. These narrowings are marked with the reference number 10. They are intended to allow patients performing self measurement to be able to see the coloured part of the eye through a mirror, on either side of the narrowing, so that it is easy for them to use this to align the measuring apparatus accurately, thus ensuring that the probe will strike the desired point on the surface of the eyeball. As can be seen, the narrowings 10 are located next to the probe 3.

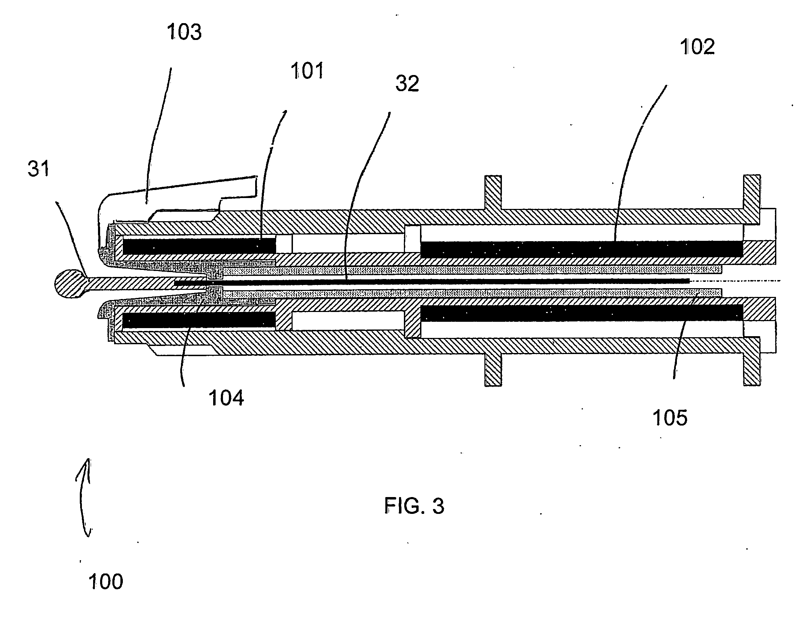

Figure 3 shows the totality, marked with the reference number 100, relating to the launch of the probe of the apparatus according to the invention, the recording of the measurement, and other similar operations. Thus, according to one well regarded embodiment, the totality 100 includes two coils 101 and 102, the frontmost of which is intended to provide the probe 3 with the necessary velocity.

The rearmost coil 102, on the other hand, is related to various measurement and settings functions.

An inner tube 105 is held in place by an inner sleeve 104, which lies around the probe and is secured by means of a separate plug 103 particularly equipped with a screw attachment. The inner sleeve and the inner tube can be changed. Naturally, the probe 3 can be changed while over time the inner sleeve and inner tube may also collect enough extraneous material for this to interfere with measurement in an apparatus with such small tolerances. As cleaning the sleeve and tube is difficult, it will then be easier to change them. If desired, the inner sleeve and the inner tube can be manufactured as a single integrated component.

According to one well regarded embodiment, the probe can be formed of a non-magnetic point part 31 and a shaft part 32 of steel or other similar material.

The point part 31 is preferably manufactured from a plastic material, for reasons of both manufacturing technique and manufacturing cost. Another characteristic of the probe is that a shoulder is formed where the point joins the shaft and is made to rest on the edges of the opening of the inner sleeve 104, through which the shaft part 32 runs. This positions the probe very precisely for the start of the measurement event. For reasons of hygiene, the point part of the probe is generally clearly outside the apparatus in the starting position.

The non-magnetic point part of the probe is in such a ratio to the magnetic shaft part that the shaft extends for a certain distance inside the coil 101, preferably, for example, almost halfway into the coil, counting from its front, as shown in Figure 3. The voltage fed to the coil induces a pushing force in the probe, causing it to move towards the eye.

Earlier, the probe had the problem that, under certain conditions, the eyelashes tended to catch on the front of the probe. The point part of the probe has now been given a very round shape, thus preventing catching.

The simplified operation of the apparatus is as follows. Power is supplied to the front coil 101, causing the probe to begin moving and to impact the eye. The probe 3 rebounds and the movements, which take place in a manner depending on the intraocular pressure, are recorded with the aid of the rear coil 102, taken to a suitable data-processing unit to be processed particularly by a microprocessor, and the result is displayed by the display device 5. The power is connected to the coil 101 for the entire duration of the measurement, so that the power is cut off only when it is intended to pull the probe back inside.

The stability of the measurement is adjusted in many ways, for example, by triggering the movement of the probe always using a constant voltage, which does not alter when the voltage of the dry cells drops. The constant voltage can be adjustable, making it possible to adjust the launch speed of the probe. The starting position of the probe 3 is also made constant in a suitable manner, for example, by having the shaft of the probe rest on its starting position in the inner sleeve 103. One way of ensuring the starting position is described above. Other ways can also be used.

Many different ways can be used to ensure that the probe remains stationary. One example is that when the power to the apparatus is switched on, the rear, measuring coil 102 holds the probe stationary by magnetic force. A function that throws the probe out after measurement, can also be added to the apparatus. This can particularly be performed manually, by pressing a button. The retaining forces need not be continuous, instead, the apparatus can include a detection function, which, for instance, which monitors the probe's position and pulls a probe, which is trying to detach itself, back into place only when necessary.

As described above, the voltage fed to the coil 101 to perform the actual measurement causes the probe to start moving. The rear coil 102 detects the speed of the probe continuously, the general principle being that the probe accelerates rapidly at the start, then, as the journey continues, a change in the voltage/current supplied to the coil reduces the acceleration to a constant, relatively low level, intended to maintain even motion. The input voltage/current of the coil 101 is on for the whole time that the probe proceeds towards the eye and impacts it, but can also be cut off at after the moment of impact.

According to yet another preferable embodiment, immediately after the impact has occurred and the measurement result has been obtained, the rear coil 102 is activated and pulls the probe back to the starting position. This ensures that the probe does not detach due to the movements of the meter or for other reasons, but can be cleanly removed from its place and thrown into the garbage.

The retraction of the probe can also be activated on the basis of time. This is because, if the probe has not struck the measurement object within a predetermined time, it can be assumed that the measurement has failed, in which case the retraction is activated.

As stated, the core of the apparatus according to the invention is a microprocessor. It is easy to use this to also perform other control operations than those referred to above. In a known manner, the microprocessor can have the task of monitoring the measurement results and making error messages according to how a measurement result differs from the values to which the system should tend, according to data recorded for the use of the microprocessor. Monitoring and calculation functions of this kind are usual in microprocessor-based devices.

As the probe is light and the velocities used are low, there is no danger of damage to the eye. The method can also be applied when the eyelid is closed. The low velocity and small mass of the probe eliminate the need to anaesthetize the eye under any measurement conditions. If necessary, the meter can be calibrated with different standards, by comparing the results with those obtained by other methods, or by using experimental methods.

As has clearly been demonstrated by the above, the apparatus according to the invention has several properties, by means of which ease of use, accuracy, and reliability can be improved and maintained. Many variations are possible while remaining within the scope of the inventive idea and the accompanying Claims.

For example, instead of the front coil 101 giving the probe 3 launching power, it is also possible alternatively to use the rear coil 102, without this altering the characteristics of the apparatus. The physical properties of the coils are always selected as required.

An alternative variation that can also be presented is one, in which the magnetic-material part of the probe, marked with the reference number 32, remains permanently inside the apparatus and is prevented from coming out, by making its rear end wider, thus preventing it from coming out, or in some other suitable manner. The front part, which attaches to the rear part 32, would then be replaceable, due to the demands of hygiene. If desired, the positioning of the components could be set up to take place from the other side of the apparatus to the probe side. In other ways the construction would correspond to that described above.

Particularly a microprocessor-based apparatus can perform many functions, which have previously been impossible. Thus it is possible to perform corrections to the measurement results obtained, in which different variables are measured and observed. Thus, the measured variables could be the impact time of the probe, the time the probe is stationary, and the rebound time, while it is also possible to observe velocities and decelerations, and make various averages and ratios, etc. of them. One correction method is such in which the intraocular pressure is corrected upwards, in proportion to the inelasticity of the impact.

## Claims

1. A method for measuring intraocular pressure with an apparatus comprising a probe (3) supported and located in a case (1), means (101, 102) for giving the probe (3) a specific velocity, means (102) for measuring and recording the velocity, means for processing data, and means (5) for displaying and controlling operations, the method comprising
feeding a voltage/current pulse to the means (101, 102) for giving the probe a specific velocity, thereby to accelerate the probe;
continuously detecting the velocity of the probe using the means (102) for measuring and recording the velocity;
on the basis of the data obtained from the means (102) for measuring and recording the velocity or alternatively, after a constant time, reducing the magnitude of the voltage/current pulse fed to the means (101 or 102) for giving the probe (3) a specific velocity in order to reduce and stabilize the acceleration of the probe (3) at a constant level;
maintaining the voltage/current pulse at least until the moment of impact of the probe with the eye;
measuring the impact of the probe with the aid of the means (102) for measuring and recording the velocity in order to calculate and obtain measurement data.

2. A method according to claim 1, **characterized in that** the probe (3) has a rear part (32) made of a magnetic material and a front part (31), attached to the rear part (32) forming a shoulder, made of non-magnetic material and with a round-shaped point, the joint between the front part (31) and the rear part (32) being located, at the start of a easurement, inside the means (101, 102) for giving the probe a specific velocity at a distance from the front of the means for measuring and recording the velocity .

3. A method according to claim 1, **characterized in that** the probe (3) is held in place, except for during a measurement event, with the aid of the means (102) for measuring and recording the velocity, when power is switched on in the apparatus.

4. A method according to claim 1, **characterized in that** the case (1) has a narrowing (10) essentially next to the probe (3) to permit parts of the eye being measured to be seen, in order to ensure accuracy.

5. A method according to claim 1, **characterized in that** a replaceable inner tube (105) is located in the apparatus, inside of which the rear part of the probe (3) is placed, and an inner sleeve (104) for holding the inner tube (105) in place.

6. A method according to claim 2, **characterized in that** the shoulder between the front part (31) and the rear part (32) of the probe (3) is placed, at the start of a measurement, against an opening in the inner sleeve (104), which receives an edge of the rear part (32) of the probe (3).

7. A method according to claim 1, **characterized by** further comprising correcting the measurement data based on an algorithm.

8. A method according to claim 1, **characterized by** further comprising correcting the measurement data according to how much kinetic energy is lost in the impact and rebound of the probe.

9. A method according to claim 1, **characterized in that** the means (101, 102) for giving the probe a specific velocity is a coil.

10. A method according to claim 1, **characterized by** further comprising retracting the probe back to a departure position within the apparatus by using a voltage fed to the means (102) for measuring and recording the velocity.

## Patentansprüche

1. Verfahren zur Messung des Augeninnendrucks mit einer Vorrichtung bestehend aus einer Sonde (3), die in einem Gehäuse (1) abgestützt und angeordnet ist, Mitteln (101, 102) zum Versetzen der Sonde (3) in eine spezifische Geschwindigkeit, Mitteln (102) zur Messung und Aufzeichnung der Geschwindigkeit, Mitteln zur Verarbeitung der Daten und Mitteln (5) zur Anzeige und Kontrolle von Funktionen, das Verfahren bestehend aus
Beaufschlagung der Mittel (101, 102) mit einer Spannung /einem Stromimpuls, um die Sonde in eine spezifische Geschwindigkeit zu versetzen, um die Sonde dadurch zu beschleunigen,
kontinuierlicher Ermittlung der Geschwindigkeit der Sonde mit Hilfe der Mittel (102) zur Messung und Aufzeichnung der Geschwindigkeit;
auf Basis der von den Mitteln (102) erhaltenen Daten zur Messung und Aufzeichnung der Geschwindigkeit oder wahlweise, nach einer konstanten Zeit, Zurücknahme des Betrags der/des den Mitteln (101 oder 102) zugeführten Spannung/Stromimpulses, um die Sonde (3) in eine spezifische Geschwindigkeit zu versetzen, um die Beschleunigung der Sonde (3) zu reduzieren und auf einem konstanten Nivea zu stabilisieren;
Aufrechterhalten der Spannung / des Stromimpulses zumindest bis zum Zeitpunkt des Aufschlags der Sonde auf dem Auge;
Messung des Aufschlags der Sonde mit Hilfe der Mittel (102) zur Messung und Aufzeichnung der Geschwindigkeit, um Messdaten zu berechnen und zu erhalten.

2. Verfahren nach Patentanspruch 1, **dadurch gekennzeichnet, dass** die Sonde (3) einen hinteren Teil (32) aus magnetischem Material und einen vorderen Teil (31) aufweist, der am hinteren Teil (32) angebracht ist und eine Schulter bildet, der aus einem nicht magnetischen Material besteht und eine rund geformte Spitze hat, wobei der Übergang zwischen dem vorderen Teil (31) und dem hinteren Teil (32) zu Beginn der Messung innerhalb der Mittel (101, 102) positioniert ist, um die Sonde in eine spezifischen Geschwindigkeit in einem Abstand von der Vorderseite der Mittel zur Messung und Aufzeichnung der Geschwindigkeit zu versetzen.

3. Verfahren nach Patentanspruch 1, **dadurch gekennzeichnet, dass** die Sonde (3), abgesehen vom Messvorgang, mit Hilfe der Mittel (102) zur Messung und Aufzeichnung der Geschwindigkeit in Position gehalten wird, wenn die Vorrichtung eingeschaltet wird.

4. Verfahren nach Patentanspruch 1, **dadurch gekennzeichnet, dass** das Gehäuse (1) eine Einengung im Wesentlich benachbart zur Sonde (3) hat, damit Teile des zu vermessenden Auges gesehen werden können, um Genauigkeit sicherzustellen.

5. Verfahren nach Patentanspruch 1, **dadurch gekennzeichnet, dass** in der Vorrichtung ein austauschbares Innenrohr (105), dessen Inneres den hinteren Teil der Sonde (3) aufnimmt, und eine Innenhülse (104) angeordnet sind, um das Innenrohr in Position zu halten.

6. Verfahren nach Patentanspruch 2, **dadurch gekennzeichnet, dass** die Schulter zwischen dem vorderen Teil (31) und dem hinteren Teil (32) der Sonde (3) zu Beginn der Messung an einer Öffnung in der Innenhülse (104) anliegt, die eine Kante des hinteren Teils (32) der Sonde (3) aufnimmt.

7. Verfahren nach Patentanspruch 1, **dadurch gekennzeichnet, dass** es des Weiteren die Korrektur der Messdaten auf Basis eines Algorithmus umfasst.

8. Verfahren nach Patentanspruch 1, **dadurch gekennzeichnet, dass** es des Weiteren die Korrektur der Messdaten davon abhängig umfasst, wie viel kinetische Energie beim Aufprall und Rückprall der Sonde verloren geht.

9. Verfahren nach Patentanspruch 1, **dadurch gekennzeichnet, dass** es sich bei den Mitteln (101, 102) zum Versetzen der Sonde in eine spezifische Geschwindigkeit um eine Spule handelt.

10. Verfahren nach Patentanspruch 1, **dadurch gekennzeichnet, dass** es des Weiteren das Zurückziehen der Sonde in eine Position innerhalb der Vorrichtung umfasst, indem die den Mitteln zugeführte Spannung zur Messung und Aufzeichnung der Geschwindigkeit benutzt wird.

## Revendications

1. Procédé destiné à mesurer la tension intra-oculaire avec un dispositif comprenant une sonde (3) soutenue et située dans un boîtier (1), des moyens (101, 102) pour donner à la sonde (3) une vélocité spécifique, un moyen (102) pour mesurer et enregistrer la vélocité, des moyens pour traiter les données et un moyen (5) pour visualiser et contrôler les opérations, le procédé comportant
l'alimentation d'une impulsion de tension/courant aux moyens (101, 102) pour donner à la sonde une vélocité spécifique et accélérer ainsi la sonde;
une détection continue de la vélocité de la sonde en utilisant le moyen (102) pour mesurer et enregistrer la vélocité;
sur la base des données obtenues par le moyen (102) pour mesurer et enregistrer la vélocité ou alternativement, après un délai constant, la réduction de l'amplitude de l'impulsion de tension/courant conduite aux moyens (101 ou 102) pour donner à la sonde (3) une vélocité spécifique afin de réduire et stabiliser l'accélération de la sonde (3) à un niveau constant;
le maintien de l'impulsion de tension/courant au moins jusqu'au moment du contact de la sonde avec l'oeil;
le mesurage du contact de la sonde à l'aide du moyen (102) pour mesurer et enregistrer la vélocité afin de calculer et d'obtenir les données de mesurage.

2. Procédé selon la revendication 1, **caractérisée en ce que** la sonde (3) une partie arrière (32) d'une matière magnétique et une partie avant (31), fixée à la partie arrière (32) formant une épaule, faite dans une matière non magnétique et avec un point de forme arrondie, le joint entre la partie avant (31) et la partie arrière (32) étant situé, au début de l'opération de mesure, à l'intérieur des moyens (101, 102) pour donner à la sonde une vélocité spécifique à une distance par rapport au devant des moyens pour mesurer et enregistrer la vélocité.

3. Procédé selon la revendication 1, **caractérisée en ce que** la sonde (3) est maintenue en place, sauf pendant une opération de mesure, à l'aide des moyens (102) pour mesurer et enregistrer la vélocité, quand le courant est branche à l'appareil.

4. Procédé selon la revendication 1, **caractérisé en ce que** le boîtier (1) a un rétrécissement (10), essentiellement après la sonde (3) pour permettre de visualiser les parties de oeil qui sont mesurées, afin de garantir une bonne précision.

5. Procède selon la revendication 1, **caractérisé en ce que** dans l'appareil il y a un tube interne (105) remplaçable, à l'intérieur duquel est placée la partie arrière de la sonde (3), et un manchon interne (104) pour maintenir le tube interne (105) en place.

6. Procédé selon la revendication 2, **caractérisé en ce que** l'épaule entre la partie avant (31) et la partie arrière (32) de la sonde (3) est placée, au début d'une opération de mesure, contre une ouverture dans le manchon interne (104), qui reçoit un bord de la partie arrière (32) de la sonde (3).

7. Procède selon la revendication 1, **caractérisé en ce qu'**il corrige encore les résultats de mesure basés sur un algorithme.

8. Procédé selon la revendication 1, **caractérisé en ce qu'**il comporte encore une correction des données de mesure selon la quantité d'énergie cinétique perdue lors du contact et du rebondissement de la sonde.

9. Procédé selon la revendication 1, **caractérisé en ce que** le moyen (101, 102) pour donner à la sonde une vélocité spécifique, est une bobine.

10. Procédé selon la revendication 1, **caractérisé en ce qu'**il comporte encore une rétraction de la sonde à sa position initiale à l'intérieur de l'appareil en utilisant une tension introduite dans le moyen (102) pour mesurer et enregistrer la vélocité.
